# EUROPEAN PATENT SPECIFICATION

(11) **EP 2 377 621 B1**
(45) Date of publication and mention of the grant of the patent: **01.10.2014**
(21) Application number: 11003109.3
(22) Date of filing: 13.04.2011
(51) Int. Cl.: B05B 7/24, A61M 3/02, B05B 1/32, B05B 1/30, B05B 1/26

(54) **Atomized cleaning device and method for atomizing current**
Zerstäubungsreinigungsvorrichtung und Verfahren zum Zerstäuben eines Stroms
Dispositif de nettoyage atomisé et procédé d'atomisation de courant

(30) Priority: 14.04.2010 CN 201010145885
(43) Date of publication of application: 19.10.2011
(60) Divisional application: 14157973.0
(73) Proprietor: HealthKare Enterprise Co., Ltd., Pei-Tou Taipeh City (TW)
(72) Inventor: Lee, Jui-Jen, Danshuei Township Taipei County 251 (TW)
(74) Representative: Chaillot, Geneviève

(56) References cited:
- DE-U- 1 828 097
- DE-U1-202007 017 437
- FR-A- 1 435 363
- US-A- 2 591 585
- US-A- 2 595 317
- US-A- 3 107 059
- US-A- 4 222 525
- US-A1- 2004 089 742
- US-A1- 2009 108 097

## Description

The present invention relates to a cleaning device and, more particularly, to an atomized cleaning device and it is also described a method for atomizing an air current.

A conventional atomized cleaning device comprises a container and a sprayer mounted on the container. The container has an inside provided with a chamber for receiving a cleaning (or medicine) liquid. An air current is delivered by an air pump and is introduced into the sprayer, and the cleaning liquid in the chamber of the container is sucked into the sprayer to mix with the air current so that the cleaning liquid is pressurized and atomized in the sprayer, and the atomized liquid is injected outward from the sprayer. However, it is necessary to increase the air supply from the air pump so as to increase the flow rate of the atomized liquid, thereby increasing the costs. In addition, after the cleaning liquid is atomized in the sprayer, the contact area between the atomized particles and the air is increased largely so that the temperature of the atomized liquid injected outward from the sprayer is decreased largely, thereby easily causing an uncomfortable sensation to a user especially when the user is cleaning the teeth. Further, when the pressure in the chamber of the container is not released or balanced, the cleaning liquid easily remains in the chamber of the container. The closest prior art reference was disclosed in U.S. Patent No. US-4-222-525-A, which disclosed an atomized cleaning device, comprising a container defining a chamber for receiving a liquid, and an opening; an atomizing module mounted on the container and having a sleeve and an air inlet pipe connected with one end of the sleeve to deliver an air current through the sleeve; a sprayer mounted in an inner space of the sleeve and having a channel and at least one slot; and at least one air hole provided on the air inlet pipe and connected to the chamber of the container to introduce a part of the air current into the chamber of the container. Other prior art references were disclosed in DE-18-28-097-U, US-2004/089742-A1, US-2-591-585-A, US-3-107-059-A, US-2009/108097-A1 and DE-20-2007-017437-U1, which teach similar atomized cleaning device.

In accordance with the present invention, there is provided an atomized cleaning device according to claim 1.

There is further described a method for atomizing an air current, comprising a first step of providing a container for receiving a liquid; a second step of providing an atomizing module which is connected to the container; a third step of providing a sprayer which is mounted in an inner space of the atomizing module; a fourth step of introducing an air current into the atomizing module; a fifth step of dividing the air current into a first partial air current and a second partial air current; a sixth step of introducing the second partial air current of the air current into the container; a seventh step of introducing the first partial air current of the air current into the sprayer; and a eighth step of sucking the liquid of the container into the sprayer to mix with the first partial air current of the air current so that the liquid is atomized in the sprayer and is sprayed outward from the sprayer.

According to the primary advantage of the present invention, when the smaller partial air current of the air current in the air inlet pipe is introduced through the air hole into the chamber to release or balance the air pressure in the chamber, the cleaning liquid in the chamber is accelerated to flow into the channel of the sprayer by suction of the larger partial air current of the air current so that the cleaning liquid is mixed with the air current and is atomized exactly and optimally to increase the flow rate of the atomized liquid largely.

According to another advantage of the present invention, the cleaning liquid in the chamber is sucked quickly by the larger partial air current flowing through the channel, and is pushed by the smaller partial air current flowing into the chamber so that the cleaning liquid is sucked exactly and completely and will not remain in the chamber.

According to a further advantage of the present invention, the flange of the sprayer is rotatable in concert with the sprayer and is movable to open, close or partially close the connection between the atomizing module and the sprayer so as to regulate the flow rate of the cleaning liquid from the chamber of the container to the sprayer.

According to a further advantage of the present invention, a user only needs to rotate the sprayer so as to regulate the flow rate of the cleaning liquid from the chamber of the container to the channel of the sprayer without needing any aid of a technician.

According to a further advantage of the present invention, the mixture proportion of the cleaning liquid and the air current can be regulated by rotation of the flange of the sprayer to regulate the temperature of the cleaning liquid so that the cleaning liquid injected from the sprayer can be controlled to have a higher temperature.

Further benefits and advantages of the present invention will become apparent after a careful reading of the detailed description with appropriate reference to the accompanying drawings.

In the drawings:
Fig. 1 is a perspective view of an atomized cleaning device in accordance with an example embodiment useful for understanding the present invention.
Fig. 2 is an exploded perspective view of the atomized cleaning device as shown in Fig. 1.
Fig. 3 is a front cross-sectional view of the atomized cleaning device as shown in Fig. 1.
Fig. 4 is a partially exploded perspective view of an atomized cleaning device in accordance with the preferred embodiment of the present invention.
Fig. 5 is a front cross-sectional assembly view of the atomized cleaning device as shown in Fig. 4.
Fig. 6 is a schematic operational view of the atomized cleaning device as shown in Fig. 5.
Fig. 7 is a front cross-sectional view of an atomized cleaning device in accordance with another example embodiment useful for understanding the present invention.
Fig. 8 is a partially exploded perspective view of an atomized cleaning device in accordance with another example embodiment useful for understanding the present invention.Fig. 9 is a partially exploded perspective view of an atomized cleaning device in accordance with another example embodiment useful for understanding the present invention.Fig. 10 is a front cross-sectional assembly view of the atomized cleaning device as shown in Fig. 9.
Fig. 11 is a schematic operational view of the atomized cleaning device as shown in Fig. 10.
Fig. 12 is a front cross-sectional view of an atomized cleaning device in accordance with another example embodiment useful for understanding the present invention.
Fig. 13 is a schematic operational view of the atomized cleaning device as shown in Fig. 12.

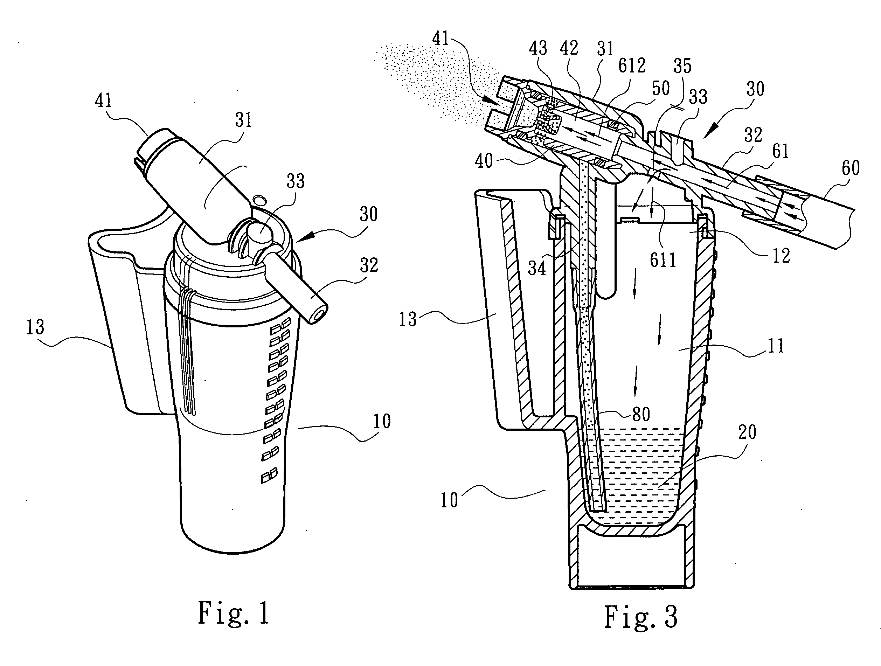

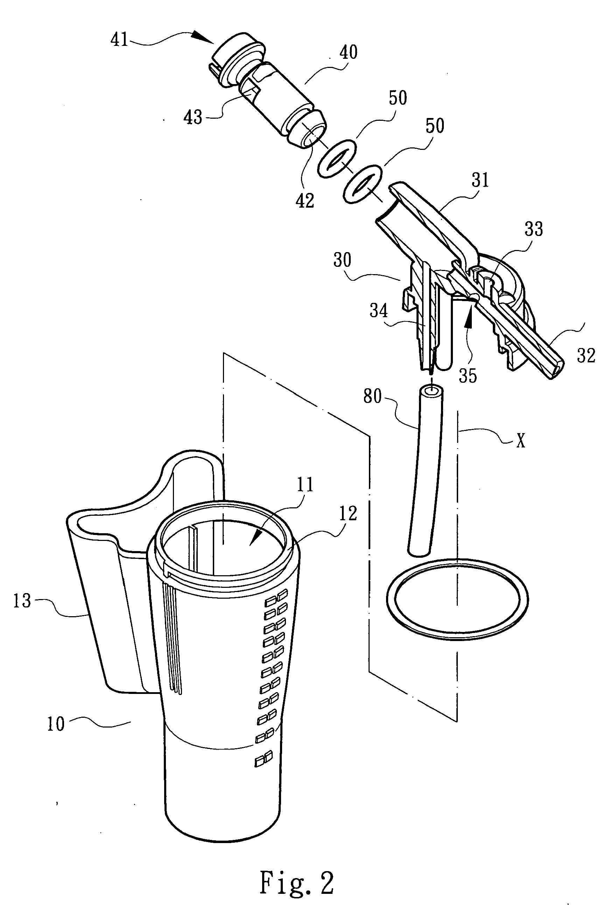

Referring to the drawings and initially to Figs. 1-3, an atomized cleaning device in accordance with an example embodiment useful for understanding the present invention comprises a container 10, an atomizing module 30 mounted on the container 10, and a sprayer 40 mounted on the atomizing module 30.

The container 10 has an inside provided with a chamber 11 for receiving a cleaning (or medicine) liquid 20 and has a top provided with an opening 12 for mounting the atomizing module 30. The container 10 has an outer wall provided with a recycling portion 13 for recycling the used cleaning liquid 20.

The atomizing module 30 is removably mounted on the opening 12 of the container 10 to seal the chamber 11 of the container 10 and has a first end provided with a sleeve 31 and a second end provided with an air inlet pipe 32 to deliver an air current 61 into the sleeve 31. The air inlet pipe 32 of the atomizing module 30 is directly connected to the sleeve 31 to directly deliver the air current 61 into the sleeve 31.

The atomizing module 30 is provided with at least one air hole 35 connected between the air inlet pipe 32 and the chamber 11 of the container 10 to connect the air inlet pipe 32 of the atomizing module 30 to the chamber 11 of the container 10 so as to introduce a part of the air current 61 of the air inlet pipe 32 into the chamber 11 of the container 10. The at least one air hole 35 of the atomizing module 30 is formed in a periphery of the air inlet pipe 32. The sleeve 31 and the air inlet pipe 32 of the atomizing module 30 are located at the same reference axis of the atomizing module 30.

The atomizing module 30 is further provided with a first conduit 34 connected between the sleeve 31 and the chamber 11 of the container 10 to connect the sleeve 31 of the atomizing module 30 to the chamber 11 of the container 10. The first conduit 34 of the atomizing module 30 is formed on a periphery of the sleeve 31 and has a first end connected to the sleeve 31 and a second end extended into and connected to the chamber 11 of the container 10. The air inlet pipe 32 of the atomizing module 30 is provided with a pressure release hole 33 to change the air drain rate or to control the air pressure in the chamber 11 of the container 10.

The sprayer 40 is rotatably and removably mounted in the sleeve 31 of the atomizing module 30 and has an inside provided with a channel 42. The channel 42 of the sprayer 40 is connected to the sleeve 31 of the atomizing module 30 and is directly connected to the air inlet pipe 32 of the atomizing module 30 through the sleeve 31 of the atomizing module 30. The sprayer 40 has a periphery provided with a plurality of slots 43 each connected between the channel 42 of the sprayer 40 and the sleeve 31 of the atomizing module 30.

The sprayer 40 has a distal end provided with a nozzle 41 which is connected to the channel 42 so that the channel 42 of the sprayer 40 is connected between the air inlet pipe 32 of the atomizing module 30 and the nozzle 41 of the sprayer 40. The nozzle 41 of the sprayer 40 allows insertion of a hand tool (not shown) so that the sprayer 40 can be rotated by the hand tool.

The atomized cleaning device further comprises a delivery pipe 60 connected with an air pump (not shown) and connected with the air inlet pipe 32 of the atomizing module 30 to deliver the air current 61 through the air inlet pipe 32 of the atomizing module 30 into the channel 42 of the sprayer 40, at least one O-ring 50 mounted on the sprayer 40 and pressed between the sprayer 40 and the sleeve 31 of the atomizing module 30 to interrupt a connection between the channel 42 of the sprayer 40 and the sleeve 31 of the atomizing module 30 and to connect the sleeve 31 of the atomizing module 30 to the slots 43 of the sprayer 40 only, and a guide pipe 80 connected with the first conduit 34 of the atomizing module 30 and extended to a bottom of the chamber 11 of the container 10 to suck the cleaning liquid 20.

As shown in Fig. 3, the cleaning liquid 20 is drawn by suction of the air current 61 so that the cleaning liquid 20 in the chamber 11 of the container 10 is introduced through the guide pipe 80 and the first conduit 34 of the atomizing module 30 into the sleeve 31 of the atomizing module 30 and is introduced through the slots 43 of the sprayer 40 into the channel 42 of the sprayer 40 to mix with the air current 61 in the channel 42 of the sprayer 40.

At this time, the air current 61 introduced into the air inlet pipe 32 of the atomizing module 30 is divided into a larger partial air current 612 and a smaller partial air current 611. In such a manner, the larger partial air current 612 of the air current 61 in the air inlet pipe 32 of the atomizing module 30 is introduced into the channel 42 of the sprayer 40 to suck, mix and pressurize the cleaning liquid 20 in the channel 42 of the sprayer 40 so that the cleaning liquid 20 is atomized and is sprayed outward from the nozzle 41 of the sprayer 40. At the same time, the smaller partial air current 611 of the air current 61 in the air inlet pipe 32 of the atomizing module 30 is introduced through the air hole 35 of the atomizing module 30 into the chamber 11 of the container 10 to release or balance the air pressure in the chamber 11 of the container 10.

Thus, when the smaller partial air current 611 is introduced through the air hole 35 into the chamber 11 to release or balance the air pressure in the chamber 11, the cleaning liquid 20 in the chamber 11 is accelerated to flow through the first conduit 34 and the slots 43 into the channel 42 by suction of the larger partial air current 612 flowing through the channel 42 so that the cleaning liquid 20 in the sprayer 40 is mixed with the larger partial air current 612 easily and quickly and is atomized exactly and optimally. In addition, the cleaning liquid 20 in the chamber 11 is sucked easily by the larger partial air current 612 flowing through the channel 42, and is pushed by the smaller partial air current 611 flowing into the chamber 11 so that the cleaning liquid 20 is sucked exactly and completely and will not remain in the chamber 11.

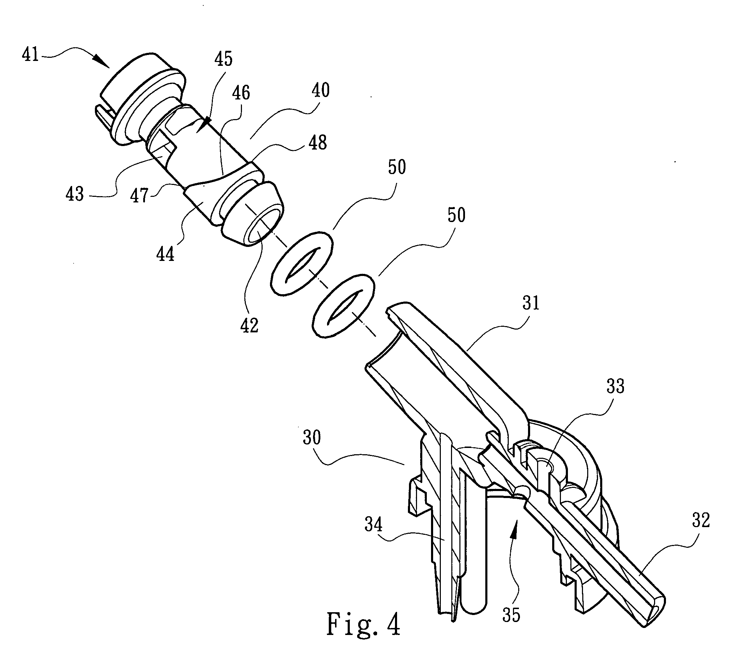

Referring to Figs. 4-6, the sprayer 40 is provided with a flange 44 that is rotatable in concert with the sprayer 40 and is movable to open, close or partially close the first conduit 34 of the atomizing module 30. The flange 44 of the sprayer 40 is formed on a surface 45 of the sprayer 40 and has a side provided with a ridge 46 which has a helical or curved shape. The ridge 46 of the sprayer 40 has a highest zone 47 and a lowest zone 48.

When in use, the flange 44 of the sprayer 40 is rotatable in concert with the sprayer 40 and is movable to open, close or partially close a connection between the first conduit 34 of the atomizing module 30 and the sleeve 31 of the atomizing module 30 so as to regulate a flow rate of the cleaning liquid 20 from the chamber 11 of the container 10 to the channel 42 of the sprayer 40. Thus, a user can rotate the sprayer 40 to easily regulate the flow rate of the cleaning liquid 20 from the chamber 11 of the container 10 to the channel 42 of the sprayer 40 without needing aid of a professional technician.

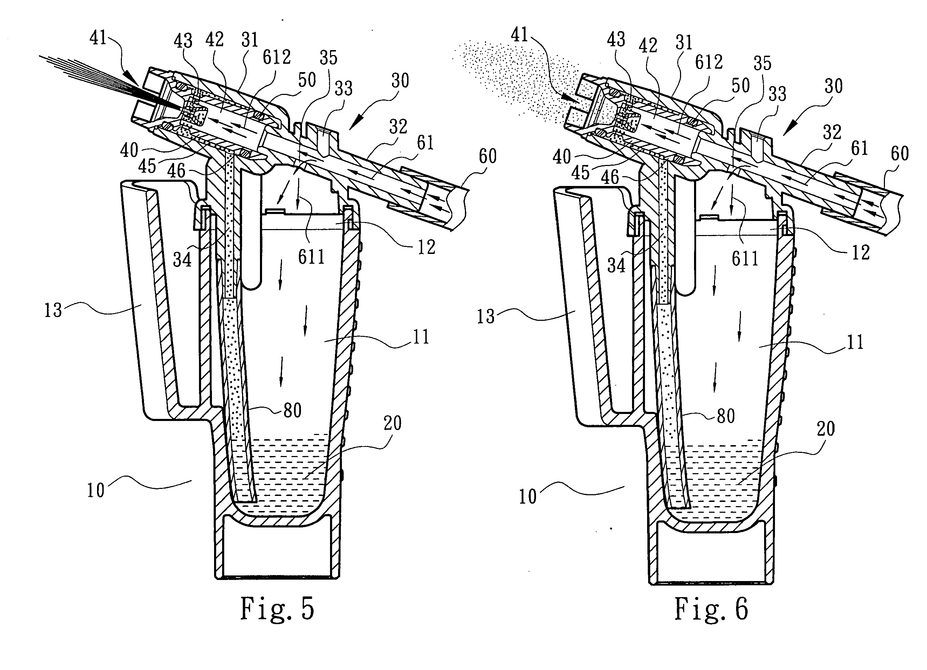

As shown in Fig. 5, the flange 44 of the sprayer 40 is rotated to fully open the first conduit 34 of the atomizing module 30 so that the flow rate of the cleaning liquid 20 from the chamber 11 of the container 10 to the channel 42 of the sprayer 40 has the maximum value. Thus, the cleaning liquid 20 injected from the sprayer 40 has a greater flow rate and can be used to clean a user's teeth. In such a manner, the cleaning liquid 20 has a smaller atomized extent to relatively reduce the mixture proportion of the cleaning liquid 20 and the air current 61 and to keep the temperature of the cleaning liquid 20 so that the cleaning liquid 20 injected from the sprayer 40 has a higher temperature.

As shown in Fig. 6, when the flange 44 of the sprayer 40 is rotated, the ridge 46 of the sprayer 40 is moved together with the highest zone 47 and the lowest zone 48 to close or locally close the first conduit 34 of the atomizing module 30 so as to regulate the flow rate of the cleaning liquid 20 from the chamber 11 of the container 10 to the channel 42 of the sprayer 40.

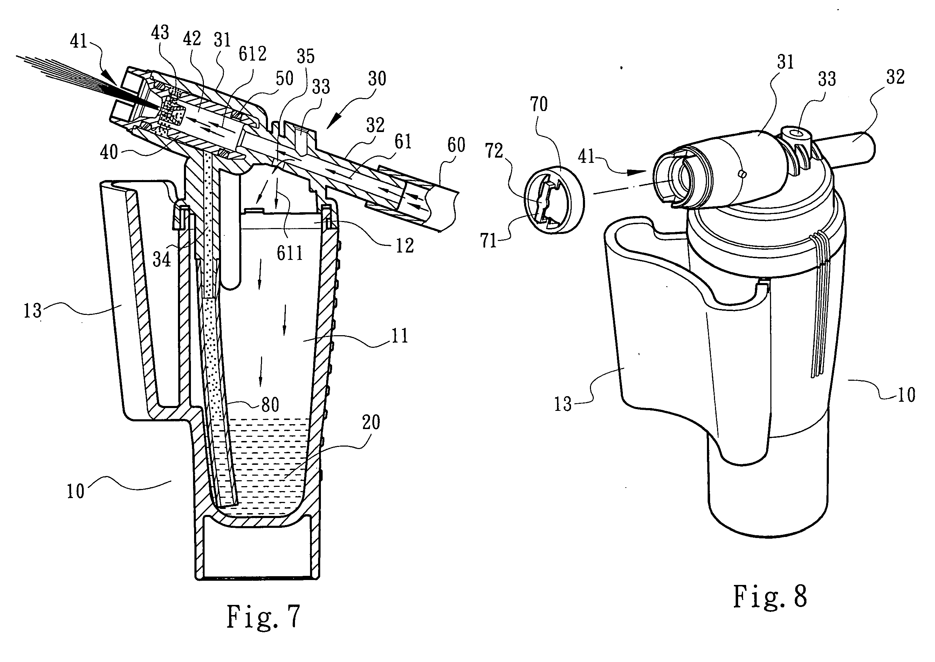

Referring to Fig. 7, the size of the guide pipe 80 can be changed to regulate the atomized status and flow rate of the atomized liquid from the sprayer 40. Preferably, the guide pipe 80 has an inner diameter greater than that of the first conduit 34 of the atomizing module 30 to increase the flow rate of the atomized liquid from the sprayer 40. Alternatively, the guide pipe 80 has an inner diameter equal or smaller than that of the first conduit 34 of the atomizing module 30. In addition, the size of the slots 43 of the sprayer 40 is changeable. Preferably, the sprayer 40 is provided with different colors or markings to indicate or distinguish different specifications and sizes of the slots 43 of the sprayer 40 so as to regulate the flow rate of the cleaning liquid 20 from the slots 43 to the channel 42 of the sprayer 40.

Referring to Fig. 8, the atomized cleaning device further comprises a buffering member 70 mounted on the nozzle 41 of the sprayer 40 to provide a disturbing effect to the nozzle 41 of the sprayer 40 to prevent the atomized air current from being injected outward from the nozzle 41 of the sprayer 40. The buffering member 70 is provided with a disturbance portion 71 which is located at a central portion of the buffering member 70 and is disposed at a path of the air current 61. The disturbance portion 71 of the buffering member 70 has a sheet or strip shape and has a surface provided with a catch portion 72.

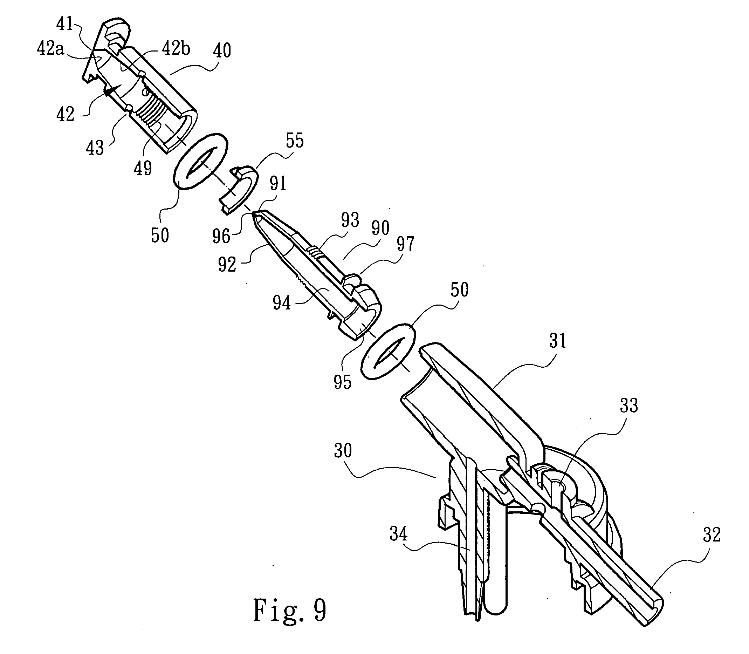

Referring to Figs. 9-11, the atomized cleaning device further comprises a regulator 90 mounted in the sprayer 40 to regulate the atomized status and flow rate of the atomized liquid from the nozzle 41 of the sprayer 40. The sprayer 40 has an inner wall provided with a first conic portion 42a, a second conic portion 42b and an inner thread 49. The regulator 90 is mounted in the channel 42 of the sprayer 40 and has an outer wall provided with a first conic section 91 facing the first conic portion 42a of the sprayer 40, a second conic section 92 facing the second conic portion 42b of the sprayer 40, and an outer thread 93 screwed onto the inner thread 49 of the sprayer 40. Thus, the regulator 90 is rotatable relative to the sprayer 40 or the sprayer 40 is rotatable relative to the regulator 90 so that the outer thread 93 of the regulator 90 is movable relative to the inner thread 49 of the sprayer 40 or the inner thread 49 of the sprayer 40 is movable relative to the outer thread 93 of the regulator 90 to change a clearance between the first conic portion 42a of the sprayer 40 and the first conic section 91 of the regulator 90 and to change a clearance between the second conic portion 42b of the sprayer 40 and the second conic section 92 of the regulator 90 so as to regulate the atomized status and flow rate of the atomized liquid from the nozzle 41 of the sprayer 40. The regulator 90 has an inside provided with a duct 94, an inlet 95 and an outlet 96. The inlet 95 of the regulator 90 is connected to the air inlet pipe 32 of the atomizing module 30. The duct 94 of the regulator 90 is connected between the inlet 95 and the outlet 96. The outlet 96 of the regulator 90 is connected to the nozzle 41 of the sprayer 40.

Thus, the air current 61 in the air inlet pipe 32 of the atomizing module 30 is introduced through the inlet 95, the duct 94 and the outlet 96 of the regulator 90 into the channel 42 and the nozzle 41 of the sprayer 40. The regulator 90 has a periphery provided with a recessed retaining shoulder 97 for mounting a gasket 55 which is mounted on the regulator 90 and is pressed between the regulator 90 and the sprayer 40. The gasket 55 is compressed by the retaining shoulder 97 of the regulator 90 via a relative rotation between the regulator 90 and the sprayer 40. Preferably, the gasket 55 is made of a resilient material.

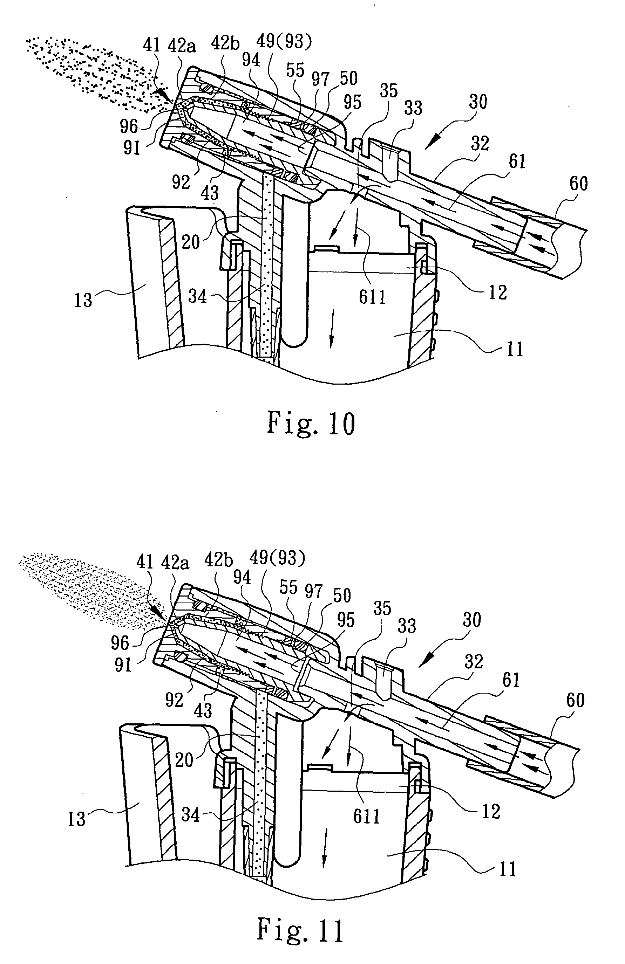

As shown in Fig. 10, when the gasket 55 is released by the relative rotation between the regulator 90 and the sprayer 40, the clearance between the first conic portion 42a of the sprayer 40 and the first conic section 91 of the regulator 90 is increased, and the clearance between the second conic portion 42b of the sprayer 40 and the second conic section 92 of the regulator 90 is increased so that the flow rate of the cleaning liquid 20 is increased, and the flow rate of the atomized liquid from the nozzle 41 of the sprayer 40 is increased.

As shown in Fig. 11, when the gasket 55 is compressed by the reverse relative rotation between the regulator 90 and the sprayer 40, the clearance between the first conic portion 42a of the sprayer 40 and the first conic section 91 of the regulator 90 is decreased, and the clearance between the second conic portion 42b of the sprayer 40 and the second conic section 92 of the regulator 90 is decreased so that the flow rate of the cleaning liquid 20 is decreased, and the flow rate of the atomized liquid from the nozzle 41 of the sprayer 40 is decreased.

Thus, the clearance between the second conic portion 42b of the sprayer 40 and the second conic section 92 of the regulator 90 is change by a first relative rotation between the regulator 90 and the sprayer 40 to provide a first-stage throttling action to regulate the flow rate of the atomized liquid from the nozzle 41 of the sprayer 40, and the clearance between the first conic portion 42a of the sprayer 40 and the first conic section 91 of the regulator 90 is change by a second relative rotation between the regulator 90 and the sprayer 40 to provide a second-stage throttling action to further regulate the flow rate of the atomized liquid from the nozzle 41 of the sprayer 40 so that the regulator 90 co-operates with the sprayer 40 to provide a two-stage throttling action.

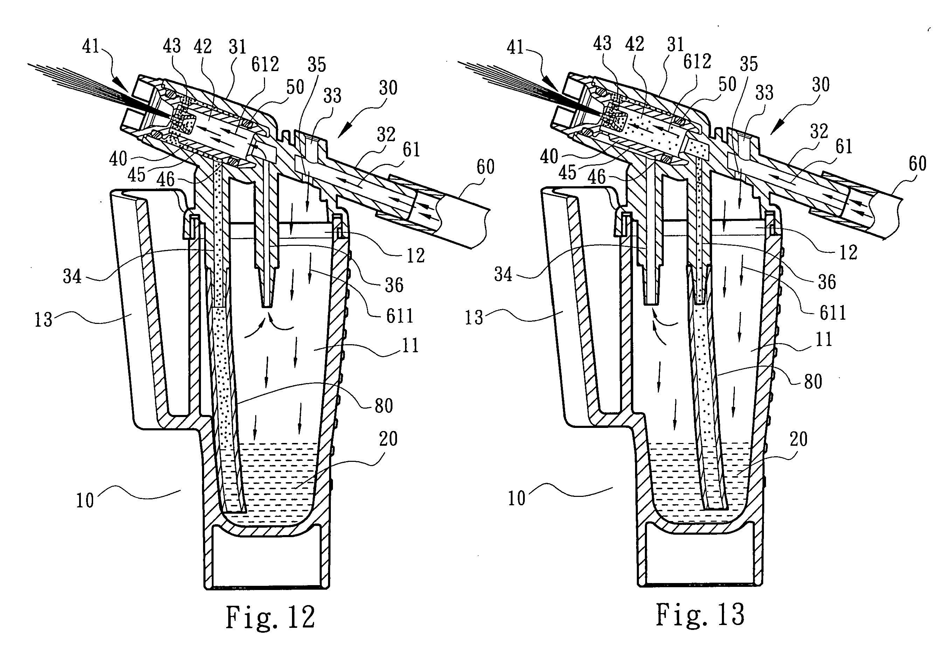

Referring to Fig. 12, the atomizing module 30 is further provided with a second conduit 36 connected between the sleeve 31 of the atomizing module 30 and the chamber 11 of the container 10 to connect the sleeve 31 of the atomizing module 30 to the chamber 11 of the container 10. The second conduit 36 of the atomizing module 30 is formed on a periphery of the sleeve 31 and has a first end connected to the sleeve 31 and a second end extended into and connected to the chamber 11 of the container 10. The second conduit 36 of the atomizing module 30 is located between the air inlet pipe 32 and the first conduit 34 and is connected to the channel 42 of the sprayer 40 through the sleeve 31 of the atomizing module 30. In practice, the air inlet pipe 32 of the atomizing module 30 is indirectly connected to the sleeve 31 of the atomizing module 30 through the air hole 35 of the atomizing module 30, the chamber 11 of the container 10 and the second conduit 36 of the atomizing module 30 to indirectly deliver the air current 61 into the sleeve 31. In addition, the channel 42 of the sprayer 40 is indirectly connected to the air inlet pipe 32 of the atomizing module 30 through the air hole 35 of the atomizing module 30, the chamber 11 of the container 10, the second conduit 36 of the atomizing module 30 and the sleeve 31 of the atomizing module 30. Thus, the larger partial air current 612 of the air current 61 is introduced through the chamber 11 of the container 10, the second conduit 36 of the atomizing module 30 and the sleeve 31 of the atomizing module 30 into the channel 42 of the sprayer 40.

Referring to Fig. 13, the air inlet pipe 32 of the atomizing module 30 is indirectly connected to the sleeve 31 of the atomizing module 30 through the air hole 35 of the atomizing module 30, the chamber 11 of the container 10 and the first conduit 34 of the atomizing module 30 to indirectly deliver the air current 61 into the sleeve 31. In addition, the atomized cleaning device further comprises a guide pipe 80 connected with the second conduit 36 of the atomizing module 30 and extended to a bottom of the chamber 11 of the container 10 to suck the cleaning liquid 20. Thus, the cleaning liquid 20 is drawn by suction of the air current 61 so that the cleaning liquid 20 in the chamber 11 of the container 10 is introduced through the guide pipe 80, the second conduit 36 of the atomizing module 30 and the sleeve 31 of the atomizing module 30 into the channel 42 of the sprayer 40 to mix with the air current 61 in the channel 42 of the sprayer 40.

Referring to Figs. 1-13, a method for atomizing an air current comprises a first step of providing a container 10 which has an inside provided with a chamber 11 for receiving a cleaning liquid 20, a second step of providing an atomizing module 30 which is mounted on the container 10 and is connected to the chamber 11 of the container 10, a third step of providing a sprayer 40 which is mounted in the atomizing module 30 and is connected to the chamber 11 of the container 10 through the atomizing module 30, a fourth step of introducing an air current 61 into the atomizing module 30, a fifth step of dividing the air current 61 into a larger partial air current 612 and a smaller partial air current 611, a sixth step of introducing the smaller partial air current 611 of the air current 61 through the atomizing module 30 into the chamber 11 of the container 10, a seventh step of introducing the larger partial air current 612 of the air current 61 into the sprayer 40, and a eighth step of sucking the cleaning liquid 20 of the chamber 11 of the container 10 through the atomizing module 30 into the sprayer 40 to mix with the larger partial air current 612 of the air current 61 so that the cleaning liquid 20 is atomized in the sprayer 40 and is sprayed outward from the sprayer 40.

The sixth step includes forming at least one air hole 35 in the atomizing module 30, and introducing the smaller partial air current 611 of the air current 61 through the air hole 35 of the atomizing module 30 into the chamber 11 of the container 10. The seventh step includes forming a channel 42 in the sprayer 40, and introducing the larger partial air current 612 of the air current 61 into the channel 42 of the sprayer 40 as shown in Fig. 3. Alternatively, the seventh step includes introducing the larger partial air current 612 of the air current 61 through the chamber 11 of the container 10 and the atomizing module 30 into the sprayer 40 as shown in Fig. 12. The eighth step includes providing the atomizing module 30 with a first conduit 34 which is connected to the chamber 11 of the container 10, and sucking the cleaning liquid 20 of the chamber 11 through the first conduit 34 of the atomizing module 30 into the sprayer 40 as shown in Fig. 3. Alternatively, the eighth step includes providing the atomizing module 30 with a first conduit 34, providing the atomizing module 30 with a second conduit 36, connecting one of the first conduit 34 and the second conduit 36 of the atomizing module 30 to the chamber 11 of the container 10, and sucking the cleaning liquid 20 of the chamber 11 through one of the first conduit 34 and the second conduit 36 of the atomizing module 30 into the sprayer 40 as shown in Figs. 12 and 13.

Accordingly, when the smaller partial air current 611 of the air current 61 in the air inlet pipe 32 is introduced through the air hole 35 into the chamber 11 to release or balance the air pressure in the chamber 11, the cleaning liquid 20 in the chamber 11 is accelerated to flow into the channel 42 of the sprayer 40 by suction of the larger partial air current 612 of the air current 61 so that the cleaning liquid 20 is mixed with the air current 61 and is atomized exactly and optimally to increase the flow rate of the atomized liquid largely. In addition, the cleaning liquid 20 in the chamber 11 is sucked quickly by the larger partial air current 612 flowing through the channel 42, and is pushed by the smaller partial air current 611 flowing into the chamber 11 so that the cleaning liquid 20 is sucked exactly and completely and will not remain in the chamber 11. Further, the flange 44 of the sprayer 40 is rotatable in concert with the sprayer 40 and is movable to open, close or partially close the connection between the atomizing module 30 and the sprayer 40 so as to regulate the flow rate of the cleaning liquid 20 from the chamber 11 of the container 10 to the sprayer 40. Further, a user only needs to rotate the sprayer 40 so as to regulate the flow rate of the cleaning liquid 20 from the chamber 11 of the container 10 to the channel 42 of the sprayer 40 without needing any aid of a technician. Further, the mixture proportion of the cleaning liquid 20 and the air current 61 can be regulated by rotation of the flange 44 of the sprayer 40 to regulate the temperature of the cleaning liquid 20 so that the cleaning liquid 20 injected from the sprayer 40 can be controlled to have a higher temperature.

## Claims

1. An atomized cleaning device, comprising:
a container (10) defining a chamber (11) for receiving a liquid (20), and an opening (12);
an atomizing module (30) mounted on the container (10) and having a sleeve (31) and an air inlet pipe (32) connected with one end of the sleeve (31) to deliver an air current (61) through the sleeve (31);
a sprayer (40) mounted in an inner space of the sleeve (31) and having a channel (42) and at least one slot (43); and
at least one air hole (35) provided on the air inlet pipe (32) and connected to the chamber (11) of the container (10) to introduce a part of the air current (61) into the chamber (11) of the container (10); wherein
the sprayer (40) has a flange (44);
the flange (44) has a helical or curved ridge (46);
the ridge (46) defines a highest zone (47) and a lowest zone (48),
**characterized in that**,
in order to regulate the flow rate of the cleaning liquid (20) from the chamber (11) of the container (10) to the channel (42) of the sprayer (40), the flange (44) of the sprayer (40) is rotated and the ridge (46) of the sprayer (40) is moved together with the highest zone (47) and the lowest zone (48) to close or locally close a first conduit (34) of the atomizing module (30) which extends through the sleeve (31) transversely and has a first end connected to the inner space of the sleeve (31) and a second end extended into the chamber (11).

2. The atomized cleaning device of claim 1, wherein the channel (42) of the sprayer (40) is connected to the air inlet pipe (32).

3. The atomized cleaning device of claim 1, wherein the atomized cleaning device further comprises a guide pipe (80) connected with the first conduit (34) which is extended into the chamber (11).

4. The atomized cleaning device of any of claims 1-3, wherein the sleeve (31) and the air inlet pipe (32) are located at the same reference axis.

5. The atomized cleaning device of any of claims 1-4, wherein
the sprayer (40) has a nozzle (41);
the atomized cleaning device further comprises a buffering member (70) mounted on the nozzle (41) of the sprayer (40);
the buffering member (70) has a disturbance portion (71).

6. The atomized cleaning device of any of claims 1-4, wherein the sprayer (40) is provided with a plurality of slots (43) extended from an exterior of the sprayer (40) into the channel (42).

7. The atomized cleaning device of any of claims 1-4, wherein
the atomized cleaning device further comprises a regulator (90) mounted in the sprayer (40);
the sprayer (40) has an inner wall provided with a first conic portion (42a), a second conic portion (42b) and an inner thread (49);
the regulator (90) is mounted in the channel (42) of the sprayer (40) and has an outer wall provided with a first conic section (91) facing the first conic portion (42a) of the sprayer (40), a second conic section (92) facing the second conic portion (42b) of the sprayer (40), and an outer thread (93) screwed onto the inner thread (49) of the sprayer (40);
the regulator (90) is rotatable and movable relative to the sprayer (40) so that the outer thread (93) of the regulator (90) is movable relative to the inner thread (49) of the sprayer (40) to change a clearance between the first conic portion (42a) of the sprayer (40) and the first conic section (91) of the regulator (90) and to change a clearance between the second conic portion (42b) of the sprayer (40) and the second conic section (92) of the regulator (90);
the regulator (90) has an inside provided with a duct (94), an inlet (95) and an outlet (96).

8. The atomized cleaning device of claim 7, wherein the regulator (90) is provided with a retaining shoulder (97) for limiting a gasket (55) which is mounted on the regulator (90).

9. The atomized cleaning device of any of claims 1-8, wherein the atomizing module (30) includes at least one first conduit (34) which transversely extends through an inner space and an exterior of a middle portion of the sleeve (31), and at least one second conduit (36) which transversely extends through an innermost portion of the inner space of the sleeve (31).

10. The atomized cleaning device of claim 9, wherein the atomized cleaning device further comprises a guide pipe (80) connected with one of the first conduit (34) and the second conduit (36).

## Patentansprüche

1. Zerstäubungsreinigungsvorrichtung, umfassend:
einen Behälter (10), der eine Kammer (11) zum Aufnehmen einer Flüssigkeit (20) und eine Öffnung (12) definiert;
ein Zerstäubungsmodul (30), das auf dem Behälter (10) befestigt ist und eine Hülse (31) sowie ein Luftzuführungsrohr (32), das mit einem Ende der Hülse (31) verbunden ist, aufweist, um einen Luftstrom (61) durch die Hülse (31) zu befördern;
einen Sprüher (40), der in einem Innenraum der Hülse (31) befestigt ist und einen Kanal (42) sowie mindestens einen Schlitz (43) aufweist; und
mindestens ein Luftloch (35), das an dem Luftzuführungsrohr (32) vorgesehen und mit der Kammer (11) des Behälters (10) verbunden ist, damit ein Teil des Luftstroms (61) in die Kammer (11) des Behälters (10) eingeleitet wird; wobei
der Sprüher (40) einen Bund (44) aufweist;
der Bund (44) eine schräge oder gebogene Kante (46) aufweist;
die Kante (46) einen höchsten Bereich (47) und einen niedrigsten Bereich (48) definiert,
**dadurch gekennzeichnet, dass**
zur Regelung des Durchsatzes der Reinigungsflüssigkeit (20) aus der Kammer (11) des Behälters (10) zum Kanal (42) des Sprühers (40) der Bund (44) des Sprühers (40) gedreht wird und die Kante (46) des Sprühers (40) zusammen mit dem höchsten Bereich (47) und dem niedrigsten Bereich (48) bewegt wird, um eine erste Röhre (34) des Zerstäubungsmoduls (30), die quer durch die Hülse (31) verläuft und ein erstes Ende aufweist, das mit dem Innenraum der Hülse (31) verbunden ist, und ein zweites Ende, das in die Kammer (11) hineinreicht, zu verschließen oder lokal zu verschließen.

2. Zerstäubungsreinigungsvorrichtung nach Anspruch 1, wobei der Kanal (42) des Sprühers (40) mit dem Luftzuführungsrohr (32) verbunden ist.

3. Zerstäubungsreinigungsvorrichtung nach Anspruch 1, wobei die Zerstäubungsreinigungsvorrichtung ferner ein Führungsrohr (80) umfasst, das mit der ersten Röhre (34) verbunden ist, die in die Kammer (11) hineinreicht.

4. Zerstäubungsreinigungsvorrichtung nach einem der Ansprüche 1 bis 3, wobei die Hülse (31) und das Luftzuführungsrohr (32) in derselben Bezugsachse liegen.

5. Zerstäubungsreinigungsvorrichtung nach einem der Ansprüche 1 bis 4, wobei
der Sprüher (40) eine Düse (41) aufweist;
die Zerstäubungsreinigungsvorrichtung ferner ein Pufferelement (70) umfasst, das an der Düse (41) des Sprühers (40) befestigt ist;
das Pufferelement (70) einen Störabschnitt (71) aufweist.

6. Zerstäubungsreinigungsvorrichtung nach einem der Ansprüche 1 bis 4, wobei der Sprüher (40) mit einer Vielzahl von Schlitzen (43) versehen ist, die von einer Außenseite des Sprühers (40) in den Kanal (42) reichen.

7. Zerstäubungsreinigungsvorrichtung nach einem der Ansprüche 1 bis 4, wobei
die Zerstäubungsreinigungsvorrichtung ferner einen Regler (90) umfasst, der in dem Sprüher (40) befestigt ist;
der Sprüher (40) eine Innenwand aufweist, die mit einem ersten konischen Abschnitt (42a), einem zweiten konischen Abschnitt (42b) und einem Innengewinde (49) versehen ist;
der Regler (90) in dem Kanal (42) des Sprühers (40) befestigt ist und eine Außenwand aufweist, die mit einem ersten konischen Abschnitt (91) versehen ist, der dem ersten konischen Abschnitt (42a) des Sprühers (40) gegenüberliegt, einem zweiten konischen Abschnitt (92), der dem zweiten konischen Abschnitt (42b) des Sprühers (40) gegenüberliegt, und einem Außengewinde (93), das auf das Innengewinde (49) des Sprühers (40) geschraubt ist;
der Regler (90) bezogen auf den Sprüher (40) so drehbar und bewegbar ist, dass das Außengewinde (93) des Reglers (90) bezogen auf das Innengewinde (49) des Sprühers (40) bewegbar ist, damit ein Abstand zwischen dem ersten konischen Abschnitt (42a) des Sprühers (40) und dem ersten konischen Abschnitt (91) des Reglers (90) verändert wird und ein Abstand zwischen dem zweiten konischen Abschnitt (42b) des Sprühers (40) und dem zweiten konischen Abschnitt (92) des Reglers (90) verändert wird;
der Regler (90) innen mit einem Durchgang (94), einem Einlass (95) und einem Auslass (96) versehen ist.

8. Zerstäubungsreinigungsvorrichtung nach Anspruch 7, wobei der Regler (90) mit einem Sicherungsansatz (97) zum Begrenzen einer Dichtung (55) versehen ist, die an dem Regler (90) befestigt ist.

9. Zerstäubungsreinigungsvorrichtung nach einem der Ansprüche 1 bis 8, wobei das Zerstäubungsmodul (30) mindestens eine erste Röhre (34) aufweist, die quer durch einen Innenraum und eine Außenseite eines mittleren Abschnitts der Hülse (31) verläuft, und mindestens eine zweite Röhre (36), die quer durch einen innersten Abschnitt des Innenraums der Hülse (31) verläuft.

10. Zerstäubungsreinigungsvorrichtung nach Anspruch 9, wobei die Zerstäubungsreinigungsvorrichtung ferner ein Führungsrohr (80) umfasst, das mit der ersten Röhre (34) oder der zweiten Röhre (36) verbunden ist.

## Revendications

1. Dispositif de nettoyage atomisé, comprenant :
un récipient (10) définissant une chambre (11), destinée à recevoir un liquide (20), et une ouverture (12) ;
un module d'atomisation (30) monté sur le récipient (10) et ayant un manchon (31) et un tuyau d'entrée d'air (32) relié à une extrémité du manchon (31) pour distribuer un courant d'air (61) à travers le manchon (31) ;
un pulvérisateur (40) monté dans un espace intérieur du manchon (31) et ayant un canal (42) et au moins une fente (43) ; et
au moins un trou d'air (35) ménagé sur le tuyau d'entrée d'air (32) et relié à la chambre (11) du récipient (10) pour introduire une partie du courant d'air (61) dans la chambre (11) du récipient (10) ; dans lequel :
le pulvérisateur (40) a un rebord (44) ;
le rebord (44) a une arête hélicoïdale ou incurvée (46) ;
l'arête (46) définit une zone la plus haute (47) et une zone la plus basse (48),
**caractérisé par le fait qu'**afin de réguler le débit du liquide de nettoyage (20) de la chambre (11) du récipient (10) au canal (42) du pulvérisateur (40), le rebord (44) du pulvérisateur (40) est tourné et l'arête (46) du pulvérisateur (40) est déplacée conjointement avec la zone la plus haute (47) et la zone la plus basse (48) pour fermer ou fermer localement un premier conduit (34) du module d'atomisation (30) qui s'étend transversalement à travers le manchon (31) et a une première extrémité reliée à l'espace intérieur du manchon (31) et une seconde extrémité étendue dans la chambre (11).

2. Dispositif de nettoyage atomisé selon la revendication 1, dans lequel le canal (42) du pulvérisateur (40) est relié au tuyau d'entrée d'air (32).

3. Dispositif de nettoyage atomisé selon la revendication 1, dans lequel le dispositif de nettoyage atomisé comprend en outre un tuyau de guidage (80) relié au premier conduit (34) qui est étendu dans la chambre (11).

4. Dispositif de nettoyage atomisé selon l'une quelconque des revendications 1 à 3, dans lequel le manchon (31) et le tuyau d'entrée d'air (32) sont situés sur le même axe de référence.

5. Dispositif de nettoyage atomisé selon l'une quelconque des revendications 1 à 4, dans lequel :
le pulvérisateur (40) a une buse (41) ;
le dispositif de nettoyage atomisé comprend en outre un élément tampon (70) monté sur la buse (41) du pulvérisateur (40) ;
l'élément tampon (70) a une partie perturbatrice (71).

6. Dispositif de nettoyage atomisé selon l'une quelconque des revendications 1 à 4, dans lequel le pulvérisateur (40) comporte une pluralité de fentes (43) s'étendant à partir d'un extérieur du pulvérisateur (40) dans le canal (42).

7. Dispositif de nettoyage atomisé selon l'une quelconque des revendications 1 à 4, dans lequel :
le dispositif de nettoyage atomisé comprend en outre un régulateur (90) monté dans le pulvérisateur (40) ;
le pulvérisateur (40) a une paroi intérieure comportant une première partie conique (42a), une seconde partie conique (42b) et un filetage intérieur (49) ;
le régulateur (90) est monté dans le canal (42) du pulvérisateur (40) et a une paroi extérieure comportant une première section conique (91) tournée vers la première partie conique (42a) du pulvérisateur (40), une seconde section conique (92) tournée vers la seconde partie conique (42b) du pulvérisateur (40), et un filetage extérieur (93) vissé sur le filetage intérieur (49) du pulvérisateur (40) ;
le régulateur (90) est apte à tourner et mobile par rapport au pulvérisateur (40) de telle sorte que le filetage extérieur (93) du régulateur (90) est mobile par rapport au filetage intérieur (49) du pulvérisateur (40), pour modifier un jeu entre la première partie conique (42a) du pulvérisateur (40) et la première section conique (91) du régulateur (90) et pour modifier un jeu entre la seconde partie conique (42b) du pulvérisateur (40) et la seconde section conique (92) du régulateur (90) ;
le régulateur (90) a un intérieur comportant un conduit (94), une entrée (95) et une sortie (96).

8. Dispositif de nettoyage atomisé selon la revendication 7, dans lequel le régulateur (90) comporte un épaulement de retenue (97) pour limiter un joint d'étanchéité (55) qui est monté sur le régulateur (90).

9. Dispositif de nettoyage atomisé selon l'une quelconque des revendications 1 à 8, dans lequel le module d'atomisation (30) comprend au moins un premier conduit (34), qui s'étend transversalement à travers un espace intérieur et un extérieur d'une partie centrale du manchon (31), et au moins un second conduit (36) qui s'étend transversalement à travers une partie la plus à l'intérieur de l'espace intérieur du manchon (31).

10. Dispositif de nettoyage atomisé selon la revendication 9, dans lequel le dispositif de nettoyage atomisé comprend en outre un tuyau de guidage (80) relié à l'un du premier conduit (34) et du second conduit (36).
